(19) Europäisches Patentamt
European Patent Office
Office européen des brevets

(11) **EP 2 464 463 B1**

(12) **EUROPÄISCHE PATENTSCHRIFT**

(45) Veröffentlichungstag und Bekanntmachung des Hinweises auf die Patenterteilung:
**09.04.2014 Patentblatt 2014/15**

(21) Anmeldenummer: **10737289.8**

(22) Anmeldetag: **22.07.2010**

(51) Int Cl.:
***B07C 5/36*** (2006.01)

(86) Internationale Anmeldenummer:
**PCT/EP2010/004491**

(87) Internationale Veröffentlichungsnummer:
**WO 2011/023269 (03.03.2011 Gazette 2011/09)**

(54) **SORTIERVERFAHREN MITTELS LUFTSTÖSSEN UND ZUGEHÖRIGE SORTIERVORRICHTUNG**

SORTING METHOD USING AIR JETS AND APPARATUS

PROCÉDÉ ET DISPOSITIF DE TRI PAR JET D'AIR

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **28.08.2009 DE 102009039211**

(43) Veröffentlichungstag der Anmeldung:
**20.06.2012 Patentblatt 2012/25**

(73) Patentinhaber: **FESTO AG & Co. KG**
**73734 Esslingen (DE)**

(72) Erfinder:
• **WIRTL, Hannes**
**86958 Schongau (DE)**
• **MAICHL, Martin**
**73084 Salach (DE)**

(74) Vertreter: **Abel, Martin**
**Patentanwälte**
**Magenbauer & Kollegen**
**Plochinger Strasse 109**
**73730 Esslingen (DE)**

(56) Entgegenhaltungen:
**DE-A1- 4 033 081 US-A- 5 628 411**

**Beschreibung**

Die Erfindung betrifft ein Verfahren zum Aussortieren von Teilen mittels Luftstößen. Ferner betrifft die Erfindung eine Sortiervorrichtung zum Aussortieren von Teilen mittels Luftstößen, mit mindestens einem Luftstoßgenerator, der ein Generatorgehäuse aufweist, in dem mindestens eine reversibel auslenkbare Membraneinheit eine mit mindestens einer Ausstoßöffnung verbundene Arbeitskammer begrenzt, wobei durch Aktivierung der Membraneinheit das Austreten eines Luftstoßes aus der Arbeitskammer durch die Ausstoßöffnung hindurch hervorrufbar ist, wobei die Membraneinheit als eine flexible Verdrängungsmembran enthaltende Luftverdrängungseinheit ausgebildet ist, die durch ihre Aktivierung im Sinne einer schlagartigen Verringerung des Volumens der Arbeitskammer zur Ausführung einer Ausstoßbewegung auslenkbar ist, so dass sie die den Luftstoß bildende Luft aktiv durch die Ausstoßöffnung hindurch impulsartig aus der Arbeitskammer verdrängt.

Eine aus der US-A-5,628,411 bekannte Sortiervorrichtung enthält einen Luftstoßgenerator mit einem Gehäuse, das eine Gehäusekammer umgrenzt, in der sich eine flexible, piezoelektrische Membraneinheit erstreckt. Die Membraneinheit begrenzt eine Arbeitskammer, die mit einer ins Freie führenden Ausstoßöffnung kommuniziert, wobei sie der inneren Mündung der Arbeitskammer gegenüberliegt. Über einen weiteren Kanal ist Druckluft in die Arbeitskammer einspeisbar, die an einem Durchtritt durch die Ausstoßöffnung gehindert ist, solange die Membraneinheit die innere Mündung der Ausstoßöffnung verschließt. Um einen austretenden Luftstoß hervorzurufen, wird die Membraneinheit durch elektrische Aktivierung ausgelenkt, so dass sie von der Mündung der Ausstoßöffnung abhebt und die unter Überdruck stehende Luft aus der Arbeitskammer ausströmen kann. Der Luftstoßgenerator hat also die Funktionalität eines Ventils, bei dem die Membraneinheit als Ventilglied fungiert, das je nach Aktivierungszustand einen Luftaustritt ermöglicht oder verhindert.

Die aus der US-A-5,628,411 bekannte Sortiervorrichtung wird für ein Verfahren zum Aussortieren von Teilen mittels Luftstößen genutzt. Herabfallende Teile, beispielsweise Reiskörner, können optisch geprüft und bei Auftreten eines Schlechtteils aussortiert werden. Zum Aussortieren wird mittels des Luftstoßgenerators ein das Schlechtteil wegblasender Luftstoß generiert. Da es hierzu eines sehr präzisen und auch relativ intensiven Luftstoßes bedarf, muss die Arbeitskammer mit unter hohem Überdruck stehender Druckluft gespeist werden. Die dafür erforderlichen Maßnahmen sind relativ aufwendig, und auch an die zuverlässige Gewährleistung der Ventilfunktion sind hohe Ansprüche gestellt, um Leckage zu vermeiden und eine daraus möglicherweise resultierende Fehlfunktion beim Aussortieren.

Aus der DE 40 30 344 A1 ist eine Sortiervorrichtung bekannt, bei der mittels elektrisch getriggerten Pulsgebern Druckstöße erzeugt werden, die über vorgeschaltete Steuerdüsen, deren Kanalausmündungen abgeschrägt sind, auf die zu sortierenden Gegenstände gerichtet werden.

Die DE 38 08 798 A1 offenbart eine Sortiervorrichtung, bei der ein Sortiervorgang durch eine kurzzeitige Verengung eines abzweigenden Kanals hervorgerufen wird, indem mittels einer piezoelektrischen Vorrichtung auf eine Stelle dieses Kanals kurze Schläge ausgeführt werden.

Die DE 20 2007 001 884 U1 beschreibt einen Schallwellengenerator zur Erzeugung von Schallwellen, die beispielsweise für therapeutische Zwecke eingesetzt werden. Durch Auslenkung einer an einer konischen Wandung anliegenden Membran werden Schallwellen bzw. Stoßwellen erzeugt, die von einer offensichtlich als Festkörper ausgebildeten Linse auf einen außerhalb der Vorrichtung liegenden Brennpunkt fokussiert werden. Bedingt durch die Auslenkung der Membran wird vermutlich auch ein Luftstoß erzeugt, der jedoch nicht den Weg zum Brennpunkt finden dürfte, da er von der Linse abgehalten wird. Die Linse kann zwar Schallwellen durchlassen, scheint jedoch nicht dafür geeignet zu sein, Luft hindurchtreten zu lassen.

Aus der WO 2006/083635 A2 ist ein Sortierer bekannt, der durch einen Piezokristall Flüssigkeit ausstoßen kann. Der Piezokristall ist in einer Kammer angeordnet, die mit der auszustoßenden Flüssigkeit gespeist wird.

Die DE-A-40 33 081 offenbart eine Sortiervorrichtung der eingangs genannten Art, bei der mittels einer in Schwingung versetzbaren Membrane ein stoßartiges Ausstoßen eines Luftstromes an einer Austrittsöffnung hervorrufbar ist.

Es ist die Aufgabe der vorliegenden Erfindung, ein einfaches und zuverlässiges Sortierverfahren vorzuschlagen sowie eine Sortiervorrichtung, die bei geringem konstruktivem Aufwand sehr zuverlässig arbeitet und sich einfach betreiben lässt.

Zur Lösung dieser Aufgabe werden bei einem Sortierverfahren der eingangs genannten Art die auf die auszusortierenden Teile gerichteten Luftstöße in Form von torusförmigen Wirbeln erzeugt.

Bei einer Sortiervorrichtung der eingangs genannten Art wird die Aufgabe dadurch gelöst, dass die Ausstoßöffnung so gestaltet ist, dass sie den austretenden Luftstoß als in sich rotierenden torusförmigen Luftwirbel (Vortex) ausbildet.

Bei der erfindungsgemäßen Sortiervorrichtung ist der Luftstoßgenerator für seinen Betrieb nicht zwingend auf unter Überdruck stehende Druckluft angewiesen, da die ausgestoßene Luft ihre Energie nicht maßgeblich aus einer externen Vorverdichtung bezieht, sondern unmittelbar aus der aktiven Verdrängung durch die zu der Ausstoßbewegung angetriebene Membraneinheit. Die schlagartige Auslenkung der Membraneinheit ruft einen intensiven Luftstoß hervor, der in der Lage ist, unerwünschte Teilchen wegzublasen und dadurch auszusortieren. Obgleich es prinzipiell möglich wäre, die Arbeitskammer mit vorkomprimierter Luft zu versorgen, hat das erfinderische Konzept den Vorteil, dass es auch ohne vorkomprimierte Luft in der Arbeitskammer funktionsfähig ist. Wird der Luftstoßgenerator ausschließlich mit nicht vorkomprimierter Luft betrieben, stellt sich ein energetisch besonders günstiger Betrieb ein, und es fällt keine Leckströmung an, die möglicherweise das Arbeitsergebnis beeinträchtigen könnte. Bei den durch den Luftstoßgenerator erzeugbaren Luftstößen handelt es sich um torusförmige Wirbel, die man jeweils auch als "Vortex" bezeichnen kann und die ein sehr zielgenaues und konzentriertes Beaufschlagen der auszusortierenden Teile ermöglichen. Durch entsprechende Gestaltung der Ausstoßöffnung ist ein in sich rotierender, bevorzugt ringförmiger Luftwirbel (Vortex) als Luftstoß generierbar, der besonders stabil und intensiv ist. Dies gelingt beispielsweise sehr einfach, wenn man die Austrittsmündung der Ausstoßöffnung scharfkantig gestaltet. Man kann sich den Luftstoß insbesondere als Torus vorstellen, bei dem die Luft innerhalb des "Ringkörpers" in sich rotiert. Das erfindungsgemäße Verfahren sieht ein Aussortieren von Teilen mittels eben solcher torusförmiger Luftwirbel vor, da sich herausgestellt hat, dass ein Luftstoß dieser Art sehr stabil und intensiv ist und somit die Möglichkeit bietet, auch bei sich mit hoher Frequenz bewegenden und einen nur geringen Abstand untereinander aufweisenden Teilen ein zuverlässiges individuelles Aussortieren bestimmter Teile zu ermöglichen.

Vorteilhafte Weiterbildungen der Erfindung gehen aus den Unteransprüchen hervor.

Bei dem erfindungsgemäßen Verfahren wird die Luft zur Erzeugung des torusförmigen Luftwirbels zweckmäßigerweise durch eine einen kreisförmigen Querschnitt aufweisende Ausstoßöffnung impulsartig hindurchgepresst. Als zweckmäßig hat es sich in diesem Zusammenhang erwiesen, den Durchmesser der bevorzugt einen kreisförmigen Querschnitt aufweisenden Ausstoßöffnung mindestens halb so groß zu wählen wie eine kleinste Abmessung des jeweils auszusortierenden Teils. Um also beispielsweise längliche Reiskörner auszusortieren, greift man zweckmäßigerweise auf eine Ausstoßöffnung zurück, deren Durchmesser mindestens den halben Querschnittsdurchmesser des Reiskornes aufweist.

Es ist des Weiteren vorteilhaft, zur Erzeugung der Luftwirbel mindestens eine eine flexible Verdrängungsmembran aufweisende Membraneinheit derart mit bevorzugt elektrischen Aktivierungsimpulsen anzusteuern, dass die daraus herrührende Auslenkung der Membraneinheit ein durch aktive Luftverdrängung hervorgerufenes impulsartigen Ausstoßen von Luft aus einer von der Membraneinheit begrenzten Arbeitskammer durch eine Ausstoßöffnung hindurch hervorruft.

Bei einer zweckmäßigen Ausgestaltung der Sortiervorrichtung sind die Membraneinheit und die mindestens eine Ausstoßöffnung derart angeordnet, dass unabhängig vom Aktivierungszustand der Membraneinheit eine stets offene Verbindung zwischen der Arbeitskammer und der Ausstoßöffnung vorliegt. Die Ausstoßöffnung wird also zu keinem Betriebszeitpunkt von der Membraneinheit verschlossen.

Vor allem bei der vorgenannten Ausführungsform ist es von Vorteil, wenn die Ausstoßöffnung in beiden Richtungen einen Luftdurchtritt ermöglicht. Sie fungiert dann nicht nur als Ausstoßöffnung, sondern zugleich als Ansaugöffnung, durch die hindurch die Membraneinheit wieder Luft in die Arbeitskammer zurücksaugt, wenn sie nach Ausführung einer Ausstoßbewegung wieder in die Grundstellung zurückkehrt. Auf diese Weise kann auf zusätzliche Öffnungen zur Lufteinspeisung in die Arbeitskammer verzichtet werden. Gleichwohl wäre es prinzipiell möglich, den Ansaugvorgang der Membraneinheit durch separate Lufteinspeisung zu unterstützen.

Wird die Ausstoßöffnung nicht als Ansaugöffnung genutzt, wird also die Arbeitskammer auf anderem Wege mit Luft nachgefüllt, könnte der Ausstoßöffnung prinzipiell auch ein Rückschlagventil zugeordnet werden, das einen Lufteintritt verhindert und mithin auch ein Einsaugen von Verunreinigungen.

Das Generatorgehäuse kann mehrere in ein und dieselbe Arbeitskammer mündende Ausstoßöffnungen aufweisen. Damit gleichwohl ein möglichst gebündelter Luftstoß generierbar ist, ist es empfehlenswert, die Ausstoßöffnungen möglichst nahe nebeneinander anzuordnen und in einer Weise, dass die Luftausstoßrichtung bei allen Ausstoßöffnungen die gleiche ist.

Die mindestens eine als Luftverdrängungseinheit fungierende Membraneinheit kann prinzipiell beliebig aktiviert werden. Beispielsweise können elektrische, fluidische oder rein mechanische Antriebskonzepte oder aus diesen Antriebskonzepten kombinierte Antriebskonzepte zur Anwendung kommen. Denkbar wäre es beispielsweise, die Bewegung der Membraneinheit durch eine Elektromagneteinrichtung oder durch impulsartige Fluidbeaufschlagung, insbesondere Druckluftbeaufschlagung hervorzurufen.

Als besonders empfehlenswertes Antriebskonzept für die Membraneinheit empfiehlt sich allerdings ein piezoelektrischer Aufbau. Hierbei basiert die Auslenkung der flexiblen Membraneinheit auf der Ausnutzung des umgekehrten piezoelektrischen Effekts. Ein derartiges Konzept ermöglicht eine sehr flache Bauform und folglich äußerst kompakte Außenabmessungen des Luftstoßgenerators. Außerdem ist die Leistungsaufnahme sehr gering, und es stellt sich eine nur sehr geringe Eigenerwärmung ein.

vorzugsweise umfasst die auf piezoelektrischem Prinzip basierende Membraneinheit eine an ihrem umlaufenden Außenrand am Generatorgehäuse abgestützte federelastische Verdrängungsmembran und mindestens einen fest mit der Verdrängungsmembran verbundenen Piezowandler. Bei elektrischer Aktivierung des Piezowandlers verformt sich die Verdrängungsmembran rechtwinkelig zu ihrer Hauptausdehnungsebene und verringert auf diese Weise das Volumen der Arbeitskammer, so dass darin enthaltene Luft verdrängt und durch die Ausstoßöffnung hindurch ins Freie ausgestoßen wird.

Die Verdrängungsmembran ist zweckmäßigerweise eine dünne, flexible Metallmembran. Sie kann bei einem piezoelektrischen Antriebsprinzip gleichzeitig als Elektrode für den Piezowandler genutzt werden.

Ein und dieselbe Arbeitskammer kann ohne weiteres von mehreren gleichzeitig und gleichsinnig aktivierbaren Membraneinheiten begrenzt sein. Auf diese Weise lässt sich das Volumen des Luftstoßes und auch die Intensität des Luftausstoßes trotz kompakter Abmessungen relevant vergrößern.

Bei einer Sortiervorrichtung besonders vorteilhaften Aufbaues weist die auf eine Kreisfläche umgerechnete Fläche der Verdrängungsmembran den fünffachen bis zwanzigfachen Durchmesser der mit einem kreisförmigen Querschnitt versehenen Ausstoßöffnung auf. Die Fläche der Verdrängungsmembran ist vorzugsweise von Hause aus eine Kreisfläche, was jedoch nicht zwingend ist. Hat die Verdrängungsmembran eine von der Kreisform abweichende Flächengestalt, ergibt sich der mit dem Durchmesser der Auslassöffnung zu vergleichende Durchmesser aus dem Durchmesser einer Kreisfläche, deren Flächeninhalt mit demjenigen der unkreisförmigen Fläche der Verdrängungsmembran übereinstimmt.

Auch für die bevorzugt einen kreisförmigen Querschnitt aufweisende Ausstoßöffnung ergibt sich ein besonders zweckmäßiges Maß für ihren Durchmesser. Dieser Öffnungsdurchmesser liegt vorzugsweise im Bereich des 0,3fachen bis 3fachen des mit dem Faktor 1/3 potenzierten, bei Aktivierung der Membraneinheit aus der Arbeitskammer verdrängten Volumens.

Das erfinderische Konzept ermöglicht in vorteilhafter Weise ein sehr flachbauendes Generatorgehäuse. Zweckmäßigerweise umschließt das Generatorgehäuse eine Gehäusekammer, deren Abmessungen in der Achsrichtung einer Hauptachse geringer sind als rechtwinklig dazu, und zwar insbesondere derart, dass die Gehäusekammer über einen rechteckigen Querschnitt verfügt. Innerhalb dieser Gehäusekammer kann die Membraneinheit mit bezüglich der Hauptachse geneigter Hauptausdehnungsebene angeordnet sein, so dass sie die Gehäusekammer in zwei Teilkammern unterteilt, deren eine die Arbeitskammer bildet und deren andere zweckmäßigerweise als Anschlusskammer fungiert, in der die für die elektrische Aktivierung notwendige elektrische Kontaktierung der Membraneinheit stattfindet.

In Verbindung mit einem rechteckigen Querschnitt der Gehäusekammer ist es besonders vorteilhaft, wenn die Membraneinheit mit diagonaler Erstreckung montiert ist. Die Membraneinheit unterteilt dann den Gehäusequerschnitt in zwei Teilkammern mit dreiecksförmig konturiertem Querschnitt.

Die mindestens eine Ausstoßöffnung ist bei einem Flachgestalt aufweisenden Generatorgehäuse zweckmäßigerweise an der Schmalseite angeordnet.

Sie ist dann insbesondere rechtwinkelig zur Achsrichtung der Hauptachse der Gehäusekammer orientiert.

Besonders bei mit Flachgestalt ausgeführter Form des Generatorgehäuses können mehrere Luftausstoßgeneratoren zur Bildung eines Generatorpaketes mit zueinander parallelen Hauptausdehnungsebenen aneinandergereiht werden.

Nachfolgend wird die Erfindung anhand der beiliegenden Zeichnung näher erläutert. In dieser zeigen:

Figur 1     in teils schematischer Darstellung eine zur Durchlüftung des erfindungsgemäßen Verfahrens geeignete erfindungsgemäße Sortiervorrichtung vorteilhaften Aufbaues mit einer im Längsschnitt abgebildeten vorteilhaften Bauform eines Luftstoßgenerators gemäß Schnittlinie I-I aus Figur 2,

Figur 2     den Luftstoßgenerator aus Figur 1 in einer perspektivischen Darstellung,

Figur 3     den Luftstoßgenerator aus Figuren 1 und 2 in einer Explosionsdarstellung,

Figur 4     ein sich aus mehreren Luftstoßgeneratoren gemäß Figur 2 zusammensetzendes Generatorpaket und

Figur 5     eine weitere Ausführungsform eines Luftstoßgenerators im Längsschnitt analog Schnittlinie I-I aus Figur 2.

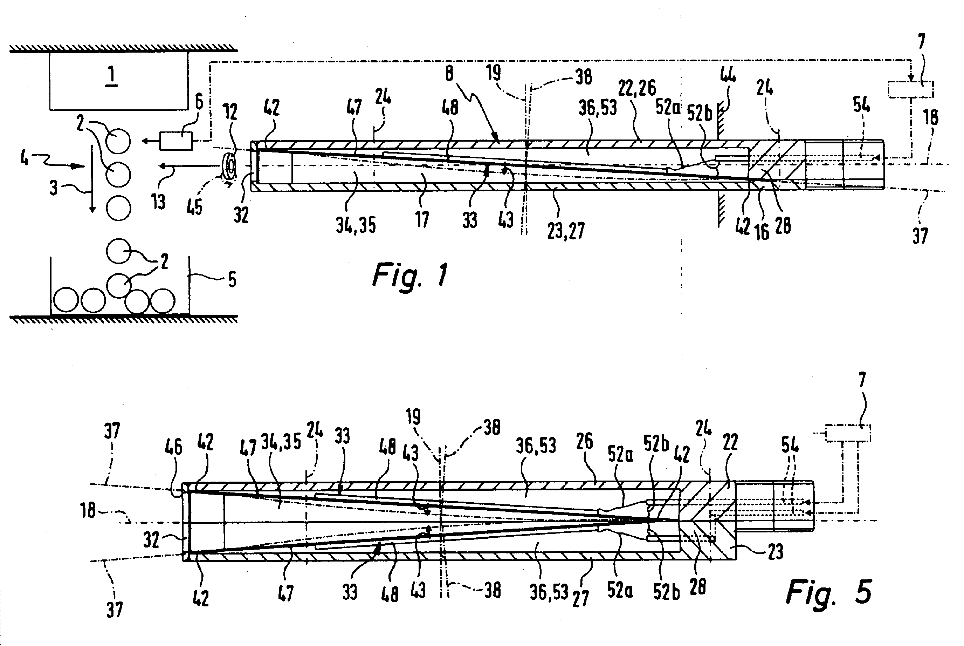

Die Figur 1 zeigt eine Sortiervorrichtung bei Anwendung des erfindungsgemäßen Sortierverfahrens. Sie verfügt über eine stationäre Ausgabeeinheit 1, aus der zu sortierende Teile 2 nach unten austreten, insbesondere schwerkraftbedingt. Die Teile 2 fallen einer Kette gleich linear aufgereiht nach unten und passieren hierbei gemäß Pfeil 3 eine Sortierzone 4. Diejenigen Teile 2, die ungestört nach unten fallen, werden von einer mit Abstand unterhalb der Ausgabeeinheit 1 angeordneten Auffangeinheit 5 aufgefangen.

Bei den zu sortierenden Teilen 2 handelt es sich um Kleinteile, beispielsweise um Lebensmittel wie Linsen oder Reiskörner. Unter solchen Teilen befinden sich überwiegend Gutteile, jedoch auch eine gewisse Anzahl von nicht den Anforderungen entsprechenden Schlechtteilen. Letztere sollen aussortiert werden.

In einer der Sortierzone 4 oberhalb vorgelagerten Überwachungszone wird durch eine Kamera oder eine sonstige Detektionseinheit 6 eine Überprüfung der Teile 2 vorgenommen und dementsprechend eine Klassifizierung ihrer Güte. Das Überwachungsergebnis wird einer elektronischen Steuereinrichtung 7 der Sortiervorrichtung zugeleitet, die an einen neben dem Fallbereich der Teile 2 platzierten Luftstoßgenerator 8 angeschlossen ist. Letzterer ist in der Lage, bei Erhalt eines entsprechenden Steuerbefehls in einer Ausstoßrichtung 13 einen schematisch angedeuteten Luftstoß 12 auszusenden.

Der Luftstoßgenerator 8 ist so orientiert, dass die Ausstoßrichtung 13 auf den Fallweg der Teile 2 gerichtet ist. Durch entsprechende Koordination mit der Steuereinrichtung 7 unter Berücksichtigung der Fallgeschwindigkeit der Teile 2 können mittels des Luftstoßgenerators 8 Luftstöße 12 derart generiert werden, dass diese auf unerwünschte Schlechtteile treffen und selbige aus dem ordentlichen Fallweg hinausblasen, so dass sie nicht in die die Gutteile aufnehmende Auffangeinheit 5 gelangen.

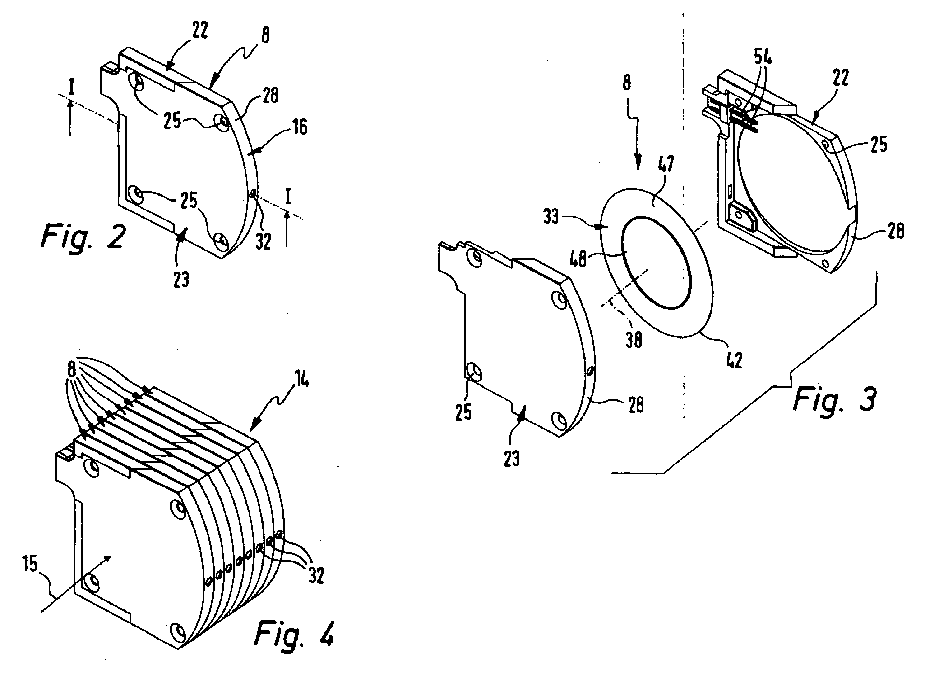

Die Sortiervorrichtung des Ausführungsbeispiels ist mit einem besonders vorteilhaft gestalteten Luftstoßgenerator 8 ausgestattet, der auch noch aus Figuren 2 und 3 ersichtlich ist. Mehrere solcher Luftstoßgeneratoren 8 können bei Bedarf gemäß Figur 4 zu einer Baugruppe paketiert werden, wobei das auf diese Weise vorliegende Generatorpaket 14 eine Mehrzahl von in einer Aufreihungsrichtung 15 aneinander gesetzten und lösbar fest miteinander verbundenen Luftstoßgeneratoren 8 beinhaltet. Die Figur 5 zeigt ein alternatives Ausführungsbeispiel eines Luftstoßgenerators 8, wobei die vorliegenden Ausführungen auch für dieses Ausführungsbeispiel gelten, sofern nichts anderes ausgesagt wird.

Der Luftstoßgenerator 8 verfügt über ein vorzugsweise eine flache Gestalt aufweisendes und insbesondere plattenförmiges Generatorgehäuse 16 mit einer Hauptausdehnungsebene 18 und einer hierzu rechtwinkeligen Hauptachse 19. Die Abmessungen in Achsrichtung der Hauptachse 19 sind erheblich geringer als in der Hauptausdehnungsebene 18.

Das Generatorgehäuse 16 begrenzt einen in seinem Innern ausgebildeten, im Folgenden als Gehäusekammer 17 bezeichneten Hohlraum. Dieser hat ebenfalls flache Gestalt mit in Achsrichtung der Hauptachse 19 geringeren Abmessungen als rechtwinkelig dazu.

Vorzugsweise besteht das Generatorgehäuse 16 aus zwei in Achsrichtung der Hauptachse 19 aneinandergesetzten ersten und zweiten Gehäusekörpern 22, 23. Diese sind exemplarisch durch schematisch angedeutete Schrauben 24, die durch Gehäuselöcher 25 hindurchgeführt sind, in vorzugsweise lösbarer Weise fest miteinander verbunden. Andere Befestigungsarten sind ebenfalls möglich, beispielsweise durch Verkleben oder Verschweißen.

Die Gehäusekammer 17 wird durch zwei sich in Achsrichtung der Hauptachse 19 mit Abstand gegenüberliegende erste und zweite Gehäusehauptwände 26, 27 sowie durch eine sich rahmenförmig um die Hauptachse 19 herum zwischen den beiden Gehäusehauptwänden 26, 27 erstreckende Seitenwand 28 umgrenzt. Die erste Gehäusehauptwand 26 ist Bestandteil des ersten Gehäusekörpers 22, die zweite Gehäusehauptwand 27 ist Bestandteil des zweiten Gehäusekörpers 23. Die Seitenwand 28 ist teils Bestandteil des ersten Gehäusekörpers 22 und teils Bestandteil des zweiten Gehäusekörpers 23. Prinzipiell wäre es auch denkbar, die Seitenwand 28 an nur einem Gehäusekörper auszubilden und den anderen Gehäusekörper lediglich nach Art eines einfachen, plattenförmigen Deckels auszubilden.

Die Seitenwand 28 ist an einer Stelle durchbrochen, wobei die Durchbrechung eine im Folgenden noch zu erläuternde Ausstoßöffnung 32 bildet. Die Ausstoßöffnung 32 stellt eine direkte Verbindung zwischen der Gehäusekammer 17 und der Umgebung des Generatorgehäuses 16, also der Atmosphäre, her. Die Ausstoßöffnung 32 hat zweckmäßigerweise einen runden, insbesondere einen kreisrunden Querschnitt.

Im Innern der Gehäusekammer 17 ist mindestens eine flexible Membraneinheit 33 aufgenommen. Beim zweiten Ausführungsbeispiel der Figur 5 befinden sich zwei Membraneinheiten 33 in der Gehäusekammer 17, das andere Ausführungsbeispiel weist in der Gehäusekammer 17 nur eine einzige Membraneinheit 33 auf.

Jede Membraneinheit 33 begrenzt eine im Innern des Generatorgehäuses 16 ausgebildete Arbeitskammer 34. Vorzugsweise ist die Arbeitskammer 34 von einer ersten zweier Teilkammern 35, 36 gebildet, in die die Gehäusekammer 17 von der jeweiligen Membraneinheit 33 gasdicht unterteilt wird. Die Membraneinheit 33 fungiert also als Trennwand zwischen einer ersten und einer zweiten Teilkammer 35, 36 der Gehäusekammer 17.

Beim Ausführungsbeispiel der Figuren 1 bis 4 verfügt die Gehäusekammer 17 auf diese Weise über insgesamt nur zwei Teilkammern 35, 36. Bei dem Ausführungsbeispiel der Figur 5 sind die hier doppelt vorhandenen Membraneinheiten 33 so angeordnet, dass jede von ihnen eine eigenständige zweite Teilkammer 36 abtrennt, jedoch nur eine einzige Arbeitskammer 34 vorhanden ist, die gleichzeitig von beiden Membraneinheiten 33 begrenzt ist.

Die Lage der Membraneinheit(en) 33 ist so gewählt, dass die Ausstoßöffnung 32 mit der Arbeitskammer 34 kommuniziert.

Die Membraneinheit 33 ist ein flaches, bevorzugt platten- oder scheibenförmiges Gebilde. Ihre zu ihrer Hauptausdehnungsebene 37 senkrechte Normalenachse ist strichpunktiert bei 38 angedeutet.

Prinzipiell könnte die Membraneinheit 33 in jedweder Ausrichtung in der Gehäusekammer 17 angeordnet sein. Zweckmäßig ist allerdings eine dahingehende Orientierung, dass sich ihre Hauptausdehnungsebene 37 quer zu der Hauptachse 19 erstreckt und ihr äußerer Randabschnitt 42 ringsum im Bereich der Seitenwand 28 zu liegen kommt. Auf diese Weise ist eine Membraneinheit 33 verwendbar, deren Grundfläche zumindest im Wesentlichen der Grundfläche der Gehäusekammer 17 im Bereich der beiden Gehäusehauptwände 26, 27 entspricht.

Die Membraneinheit 33 ist im Bereich ihres äußeren Randabschnittes 42 gasdicht an der Wandung des Generatorgehäuses 16 fixiert. Sie kann beispielsweise mittels eines elastischen Klebers oder unter Verwendung einer elastischen Dichtmasse am Generatorgehäuse 16 gehalten sein. Mittels von der elektronischen Steuereinrichtung 7 gelieferten elektrischen Aktivierungsimpulsen kann die Membraneinheit 33 derart angesteuert werden, dass sie aus einer im deaktivierten Zustand eingenommenen, im Wesentlichen ebenen Ausgangsstellung - in Figuren 1 und 5 in durchgezogenen Linien gezeigt - unter Ausführung einer durch einen Pfeil angedeuteten Ausstoßbewegung 43 in eine als strichpunktierte Linie angedeutete aktivierte Stellung ausgelenkt wird. Anschließend kehrt die Membraneinheit 33 im Rahmen einer Rückstellbewegung wieder in die Ausgangsstellung zurück. Es findet also bei Betätigung der Membraneinheit 33 ein reversibler, elastischer Verformungsvorgang statt.

Aufgrund ihrer Verformung verringert die Membraneinheit 33 bei der Ausstoßbewegung 43 das Volumen der Arbeitskammer 34. Dies hat zur Folge, dass Luft aus der Arbeitskammer 34 durch die Ausstoßöffnung 32 hindurch ins Freie gedrückt wird. Aufgrund des Umstandes, dass die Membraneinheit 33 schlagartig verformt wird, äußert sich dies in dem schon erwähnten Luftstoß 12.

Je nachdem, mit welcher Frequenz die Membraneinheit 33 aktiviert und deaktiviert wird, kann die Erzeugungsfrequenz der Luftstöße 12 beeinflusst werden. Bei einer sehr hohen Erregerfrequenz lässt sich so bei Bedarf auch ein quasi kontinuierlicher, zielgerichteter Luftstrahl aus einer Vielzahl aneinandergereihter Luftstöße 12 hervorrufen.

Ersichtlich fungiert die Membraneinheit 33 als Luftverdrängungseinheit, die bei ihrer Ausstoßbewegung 43 aktiv Luft durch die Ausstoßöffnung 32 hindurch aus der Arbeitskammer verdrängt.

Die Ausstoßöffnung 32 verfügt zweckmäßigerweise über einen kreisförmigen Querschnitt. Ihr Durchmesser "D" errechnet sich vorzugsweise aus der Formel

$$D = a \cdot V^{1/3},$$

wobei "a" ein Zahlenwert zwischen 0,3 und 3 ist, einschließlich der Bereichsgrenzen, und wobei "V" das Volumen ist, das von der Membraneinheit 33 bei ihrer Aktivierung aus der Arbeitskammer 34 verdrängt wird.

Der Luftstoßgenerator 8 ist so angeordnet und unter Verwendung von schematisch angedeuteten Befestigungsmitteln 44 so fixiert, dass die Ausstoßöffnung 32 zu den herabfallenden Teilen 2 weist. Die Orientierung der Ausstoßöffnung 32 gibt also die Ausstoßrichtung 13 vor.

Vorzugsweise ist die Membraneinheit 33 so im Generatorgehäuse 16 angeordnet, dass ihr Betätigungszustand keine Auswirkungen auf die Größe des offenen Querschnittes der Ausstoßöffnung 32 hat. Es liegt also eine ständig offene Verbindung zwischen der Arbeitskammer 34 und der Auslassöffnung 32 sowie der Umgebung des Generatorgehäuses 16 vor.

Auch ansonsten sind zweckmäßigerweise keine Mittel vorhanden, die ein wenn auch nur zeitweiliges Verschließen der Ausstoßöffnung 32 bewirken. Auf diese Weise übernimmt die Ausstoßöffnung 32 die zusätzliche Funktion einer Ansaugöffnung, wenn die Membraneinheit 33 ihre Rückstellbewegung ausführt. Die Membraneinheit 33 kann in diesem Fall vergleichbar einem Pumpenelement arbeiten, das Umgebungsluft durch die Ausstoßöffnung 32 hindurch in die Arbeitskammer 34 einsaugt. Dadurch wird die Arbeitskammer 34 ohne Fremdunterstützung selbsttätig wieder mit Luft befüllt, und es kann sich ohne Verzögerung die nächste Ausstoßbewegung 43 wirksam anschließen.

Zur Unterstützung des Luftnachfüllvorganges könnte die Wandung des Generatorgehäuses 16 mindestens eine zusätzlich zu der Ausstoßöffnung 32 vorhandene Zuströmöffnung aufweisen, über die Druckluft eingespeist wird. Dies würde es auch ermöglichen, der Ausstoßöffnung 32 ein Steuerventil zuzuordnen, insbesondere ein Rückschlagventil, das lediglich einen Luftaustritt zulässt, nicht jedoch einen Lufteintritt. Der Vorteil eines solchen Ventils bestünde insbesondere auch in der Funktionalität als Barriere gegen einen Eintritt von Verunreinigungen. Die beispielhafte Variante ohne Fremdluftzufuhr hat allerdings den Vorteil, dass vor Ort keine Luftversorgung benötigt wird und sich der Luftstoßgenerator 8 allein mit elektrischer Energie betreiben lässt.

Beide Ausführungsbeispiele sind mit einer einzigen Ausstoßöffnung 32 ausgestattet. Prinzipiell könnten allerdings auch mehrere, insbesondere auf engstem Raum verteilt angeordnete Ausstoßöffnungen 32 kleineren Querschnittes vorgesehen werden.

Besonders effektiv ist die Sortiervorrichtung aufgrund des Umstandes, dass der durch die Ausstoßöffnung 32 hindurch austretende Luftstoß ein in sich rotierender, torusförmiger Luftwirbel ist, wie dies in Figur 1 bei 45 durch einen Pfeil illustriert ist. Dadurch ergeben sich sehr stabile und präzise Luftimpulse. Ein solcher Luftwirbel wird auch als "Vortex" bezeichnet.

Eine sehr einfache Methode zur Generierung der Luftwirbel bzw. Vortexe wird darin gesehen, die Austrittsmündung 46 der Ausstoßöffnung 32 im Übergangsbereich zur Außenfläche des Generatorgehäuses 16 scharfkantig auszubilden.

Für die Membraneinheit 33 empfiehlt sich wegen der dadurch möglichen flachen Bauweise insbesondere ein piezoelektrischer Aufbau. Die gewünschte Auslenkung der Membraneinheit 33 basiert hier auf der Ausnutzung des umgekehrten piezoelektrischen Effekts. Andere Bauformen sind jedoch prinzipiell ebenfalls möglich. Beispielsweise könnte man die Membraneinheit auf andere Art elektrisch aktiviert auslenken, zum Beispiel durch elektromagnetisch generierte Betätigungskräfte. Auch eine rein mechanische und/oder durch Fluidkraft hervorgerufene Betätigung wäre realisierbar.

Bei beiden Ausführungsbeispielen verfügt die jeweilige Membraneinheit 33 über einen piezoelektrischen Aufbau. Vorzugsweise ist hierzu eine den äußeren Randabschnitt 42 bildende flexible, insbesondere federelastische Verdrängungsmembran 47 vorhanden, die auf einer ihrer großflächigen Seiten im zentralen Bereich mit einem Piezowandler 48 bestückt ist. Die Verdrängungsmembran 47 hat vorzugsweise eine kreisförmige Außenkontur, ebenso der Piezowandler 48. Beide Komponenten sind also insbesondere scheibenförmig ausgebildet und konzentrisch zueinander angeordnet.

Wird an den Piezowandler 48 über erste und zweite Kontaktelemente 52a, 52b eine Ansteuerspannung angelegt, zieht er sich bei gleichzeitiger Verdickung diametral zusammen und bewirkt ein Auswölben der großflächig mit ihm fest verbundenen Verdrängungsmembran 47. Dies äußert sich in der schon erwähnten Ausstoßbewegung 43.

Wird der Piezowandler 48 wieder entladen, kehrt die Verdrängungsmembran 47 aufgrund ihrer federelastischen Eigenschaften wieder in die Ausgangsstellung zurück. Jede Auslassbewegung 43 ruft einen Luftstoß 12 hervor.

Die auf eine Kreisfläche umgerechnete Fläche der Verdrängungsmembran 47 weist zweckmäßigerweise den fünffachen bis zwanzigfachen Durchmesser der einen kreisförmigen Querschnitt aufweisenden Ausstoßöffnung 32 auf. Ist die Verdrängungsmembran 47 unmittelbar kreisförmig gestaltet, entspricht also ihr Durchmesser vorzugsweise dem 5- bis 20fachen der Ausstoßöffnung 32. Hat die Verdrängungsmembran eine unkreisförmige Gestalt, entspricht der mit dem Durchmesser der Ausstoßöffnung zu vergleichende Durchmesser dem Durchmesser einer Kreisfläche, deren Flächeninhalt demjenigen der unkreisförmig konturierten Fläche der Verdrängungsmembran 47 entspricht.

Diese zweckmäßige Dimensionierung ist unabhängig davon, auf welchem Aktivierungsprinzip die Membraneinheit basiert und gilt also insbesondere auch dann, wenn die Membraneinheit keinen Piezowandler aufweist.

Im übrigen ist es zweckmäßig, den Durchmesser der bevorzugt einen kreisförmigen Querschnitt aufweisenden Ausstoßöffnung 32 mindestens halb so groß zu wählen wie die kleinste Abmessung der zu sortierenden Teile. Wenn also wie beim Ausführungsbeispiel die zu sortierenden Teil 2 kugelförmig gestaltet sind, sollte der Durchmesser der Ausstoßöffnung 32 mindestens dem Kugelradius entsprechen.

Besteht die Verdrängungsmembran 47 wie beim Ausführungsbeispiel aus Metall, kann sie unmittelbar die Funktion der einen Elektrode des Piezowandlers 48 übernehmen. Das eine Kontaktelement 52b kann deshalb direkt an der Verdrängungsmembran 47 angebracht werden. Die zweite Elektrode befindet sich zweckmäßigerweise auf der der Verdrängungsmembran 47 entgegengesetzten Rückseite des Piezowandlers 48. Mit ihr ist das andere Kontaktelement 52a verbunden.

Der Piezowandler 48 sitzt zweckmäßigerweise auf derjenigen Seite der Verdrängungsmembran 47, die der zweiten Teilkammer 36 zugewandt ist. Somit kann die zweite Teilkammer 36 als Anschlusskammer 53 genutzt werden, in der sich alle bezüglich der Membraneinheit 33 im Innern des Generatorgehäuses 16 zu treffenden Anschlussmaßnahmen konzentrieren. Die Kontaktelemente 52a, 52b sind innerhalb der Anschlusskammer 53 mit der Membraneinheit 33 elektrisch kontaktiert.

Auch wenn kein piezoelektrisches Antriebsprinzip für die Aktivierung der Membraneinheit 33 eingesetzt wird, kann die zweite Teilkammer 36 in vorteilhafter Weise für Maßnahmen genutzt werden, die dem Antreiben bzw. Aktivieren der Membraneinheit 33 dienen. So könnte die zweite Teilkammer 36 im Falle eines fluidischen Antriebskonzepts dafür genutzt werden, eine impulsartige Fluidbeaufschlagung der Membraneinheit 33 hervorzurufen. In diesem Falle würde in die zweite Teilkammer 36 mindestens ein Steuerkanal einmünden, über den das Betätigungsfluid zuführbar und abführbar ist.

Für den Anschluss der elektronischen Steuereinrichtung 7 sind ein oder mehrere, die Wandung des Generatorgehäuses 16 durchsetzende Anschlusskontakte 54 vorgesehen, die mit den oben erwähnten Kontaktelementen 52a, 52b kontaktiert sind, welche Letzteren flexibel sind, damit sie die Bewegungen der Membraneinheit 33 schadlos mitmachen können.

Aus Figuren 1 und 5 ist gut ersichtlich, dass die Abmessungen der Gehäusekammer 17, wie im Übrigen auch diejenigen des Generatorgehäuses 16 insgesamt, in der Achsrichtung der Hauptachse 19 zweckmäßigerweise geringer sind als in der dazu rechtwinkeligen Richtung, also in der Ausdehnungsrichtung der Hauptausdehnungsebene 18. Daraus resultiert die schon erwähnte und angestrebte flache Bauweise, und zwar insbesondere derart, dass die Gehäusekammer 17 in einer von der Hauptachse 19 und der dazu rechtwinkeligen Querachse aufgespannten Ebene einen rechteckigen Querschnitt aufweist. Die kürzeren Seiten dieses rechteckigen Querschnittes - beim Ausführungsbeispiel von der Seitenwand 28 gebildet - verlaufen parallel zu der Hauptachse 19, die längeren Seiten parallel zu der Hauptausdehnungsebene 18.

In Verbindung damit ergibt sich eine optimale Ausrichtung der Membraneinheit 33, wenn deren Hauptausdehnungsebene 37 bezüglich der Hauptachse 19 des Generatorgehäuses 16 geneigt ist. Die oben erwähnte Normalenachse 38 ist dann nicht parallel zu der Hauptachse 19, sondern nimmt diesbezüglich einen Winkel ein.

Ist die Gehäusekammer 17 mit nur einer Membraneinheit 33 bestückt, empfiehlt sich eine dahingehende Anordnung, dass sich die Membraneinheit 33 zwischen zwei sich diagonal gegenüberliegenden Eckbereichen des rechteckigen Querschnittes der Gehäusekammer 17 erstreckt (Figur 1). Der Querschnitt der Gehäusekammer 17 wird dadurch in zwei Teilkammern 35, 36 mit jeweils rechtwinkeliger Dreiecksform unterteilt, deren Querschnitts-Hypotenusen mit der Hauptausdehnungsebene 37 der Membraneinheit 33 zusammenfallen. Die Ausstoßöffnung 32 ist quer zur Hauptachse 19 orientiert.

Sind gemäß Figur 5 in der Gehäusekammer 17 gleichzeitig zwei Membraneinheiten 33 untergebracht, empfiehlt sich eine Ausrichtung mit einander entgegengesetzten Neigungen. Im Bereich der Ausstoßöffnung 32 sind die beiden sich in Achsrichtung der Hauptachse 19 gegenüberliegenden Membraneinheiten 33 am weitesten voneinander entfernt, um ausgehend von dort in Richtung zur entgegengesetzten Rückseite der Gehäusekammer 17 aufeinander zu zu laufen. Somit ergibt sich zwischen den beiden Membraneinheiten 33 eine Arbeitskammer 34 mit dreieckigem, gleichschenkeligem Querschnitt, wobei die gleichlangen Dreiecksschenkel von je einer der Membraneinheiten 33 definiert werden. Die beiden zweiten Teilkammern 36 fungieren jeweils als Anschlusskammer 53 zur elektrischen Kontaktierung des zugeordneten Piezowandlers 48.

Die elektrische Aktivierung der beiden Membraneinheiten 33 erfolgt vorzugsweise stets gleichzeitig und gleichsinnig, so dass beide Membraneinheiten 33 gleichzeitig zu der Auslassbewegung 43 angetrieben werden und zur Erzeugung des Luftstoßes 12 eine größere Menge an Luft verdrängt wird als bei einer Bauform mit nur einer Membraneinheit 33.

Die flache Bauform der Generatorgehäuse 16 prädestiniert den Luftstoßgenerator 8 zur Paketierung gemäß Figur 4. Die Luftstoßgeneratoren 8 sind hier mit zueinander parallelen Hauptausdehnungsebenen 18 aneinandergereiht und können beispielsweise unter Nutzung der miteinander fluchtenden Schraubenlöcher 25 zu dem Generatorpaket 14 zusammengespannt werden.

Zusammengefasst besteht unter anderem ein wesentlicher Vorteil der Ausführungsbeispiele darin, dass sich Luftanschlüsse für die Zufuhr von Fremdluft erübrigen, weil der Luftimpuls jeweils selbständig erzeugt wird. Der Luftstoßgenerator arbeitet reibungsfrei und ist damit extrem schnell. Es sind sehr flache Abmessungen möglich, beispielsweise mit einer Bauhöhe von nur 4 mm in Achsrichtung der Hauptachse 19. Beim piezoelektrischen Betrieb wird eine nur geringe Ansteuerleistung benötigt, so dass die Eigenerwärmung auf niedrigem Niveau bleibt. Werden die Luftstöße 12 als Wirbel erzeugt, ergeben sich sehr präzise und intensive Luftstöße. Nach entsprechender Vormontage kann der gesamte Luftstoßgenerator 8 aus im Wesentlichen nur drei oder vier Teilen zusammengefügt werden, den beiden Gehäusekörpern 22, 23 und einer oder zwei Membraneinheit(en) 33.

**Patentansprüche**

1. Verfahren zum Aussortieren von Teilen (2) mittels Luftstößen, **dadurch gekennzeichnet, dass** man die auf die auszusortierenden Teile (2) gerichteten Luftstöße in Form von torusförmigen Wirbeln erzeugt.

2. Verfahren nach Anspruch 1, **dadurch gekennzeichnet, dass** man die Luft zur Erzeugung der wirbelförmigen Luftstöße durch eine einen kreisförmigen Querschnitt aufweisende Ausstoßöffnung (32) impulsartig hindurchpresst.

3. Verfahren nach Anspruch 2, **dadurch gekennzeichnet, dass** der Durchmesser der Ausstoßöffnung (32) mindestens halb so groß ist wie eine kleinste Abmessung des durch Beaufschlagung mittels eines Luftstoßes jeweils auszusortierenden Teils (2).

4. Verfahren nach einem der Ansprüche 1 bis 3, **dadurch gekennzeichnet, dass** man mindestens eine eine flexible Verdrängungsmembran (47) aufweisende Membraneinheit (33) derart mit insbesondere elektrischen Aktivierungsimpulsen ansteuert, dass ihre daraus herrührende Auslenkung ein durch aktive Luftverdrängung hervorgerufenes impulsartiges Ausstoßen von Luft aus einer Arbeitskammer (34) durch eine Ausstoßöffnung (32) hindurch bewirkt.

5. Sortiervorrichtung zum Aussortieren von Teilen (2) mittels Luftstößen, mit mindestens einem Luftstoßgenerator (8), der ein Generatorgehäuse (16) aufweist, in dem mindestens eine reversibel auslenkbare Membraneinheit (33) eine mit mindestens einer Ausstoßöffnung (32) verbundene Arbeitskammer (34) begrenzt, wobei durch Aktivierung der Membraneinheit (33) das Austreten eines Luftstoßes aus der Arbeitskammer (34) durch die Ausstoßöffnung (32) hindurch hervorrufbar ist, wobei die Membraneinheit (33) eine eine flexible Verdrängungsmembran (47) enthaltende Luftverdrängungseinheit bildet, die durch ihre Aktivierung im Sinne einer schlagartigen Verringerung des Volumens der Arbeitskammer (34) zur Ausführung einer Ausstoßbewegung (43) auslenkbar ist, so dass sie die den Luftstoß bildende Luft aktiv durch die Ausstoßöffnung (32) hindurch impulsartig aus der Arbeitskammer (34) verdrängt, **dadurch gekennzeichnet, dass** die Ausstoßöffnung (32) so gestaltet ist, dass sie den austretenden Luftstoß als in sich rotierenden torusförmigen Luftwirbel (Vortex) ausbildet.

6. Sortiervorrichtung nach Anspruch 5, **dadurch gekennzeichnet, dass** die Membraneinheit (33) und die Ausstoßöffnung (32) derart angeordnet sind, dass unabhängig vom Aktivierungszustand der Membraneinheit (33) eine stets offene Verbindung zwischen der Arbeitskammer (34) und der Ausstoßöffnung (32) vorliegt.

7. Sortiervorrichtung nach Anspruch 5 oder 6, **dadurch gekennzeichnet, dass** die Ausstoßöffnung (32) in beiden Richtungen einen Luftdurchtritt ermöglicht und zugleich eine Ansaugöffnung bildet, durch die hindurch Luft von außen in die Arbeitskammer (34) einströmt oder eingesaugt wird, wenn die Membraneinheit (33) eine der Ausstoßbewegung (34) entgegengesetzte Rückstellbewegung ausführt.

8. Sortiervorrichtung nach einem der Ansprüche 5 bis 7, **dadurch gekennzeichnet, dass** genau eine in die Arbeitskammer (34) mündende Ausstoßöffnung (32) vorhanden ist.

9. Sortiervorrichtung nach einem der Ansprüche 5 bis 8, **dadurch gekennzeichnet, dass** die Ausstoßöffnung (32) eine scharfkantige Austrittsmündung (46) aufweist.

10. Sortiervorrichtung nach einem der Ansprüche 5 bis 9, **dadurch gekennzeichnet, dass** die mindestens eine Membraneinheit (33) elektrisch aktivierbar ausgebildet ist, wobei sie zweckmäßigerweise einen piezoelektrischen Aufbau besitzt.

11. Sortiervorrichtung nach Anspruch 10, **dadurch gekennzeichnet, dass** die Membraneinheit (33) eine mit ihrem äußeren Randabschnitt (42) am Generatorgehäuse (16) abgestützte federelastische Verdrängungsmembran (47) sowie mindestens einen fest mit der Verdrängungsmembran (47) verbundenen Piezowandler (48) aufweist, wobei die Verdrängungsmembran (47) vorzugsweise aus Metall besteht und zweckmäßigerweise zugleich eine Elektrode des Piezowandlers (48) bildet.

12. Sortiervorrichtung nach einem der Ansprüche 5 bis 11, **dadurch gekennzeichnet, dass** die Verdrängungsmembran (47) mit schräger Ausrichtung und insbesondere diagonal im Innern einer Gehäusekammer (17) des Generatorgehäuses (16) angeordnet ist, wobei sie die Gehäusekammer (17) in eine die Arbeitskammer (34) bildende erste Teilkammer (35) sowie eine zweite Teilkammer (36) gasdicht unterteilt, wobei die zweite Teilkammer (36) zweckmäßigerweise für dem Antreiben der Membraneinheit (33) dienende Maßnahmen genutzt ist.

13. Sortiervorrichtung nach einem der Ansprüche 5 bis 12, **dadurch gekennzeichnet, dass** das Generatorgehäuse (16) eine Gehäusekammer (17) umschließt, deren Abmessungen in der Achsrichtung einer Hauptachse (19) geringer sind als in der dazu rechtwinkeligen Richtung, wobei die Membraneinheit (33) in der Gehäusekammer (17) mit bezüglich der Hauptachse (19) geneigter Hauptausdehnungsebene (37) angeordnet ist und die Gehäusekammer (17) in zwei Teilkammern (35, 36) unterteilt, von denen eine die Arbeitskammer (34) bildet, wobei die Gehäusekammer (17) zweckmäßigerweise einen rechteckigen Querschnitt aufweist, dessen kürzere Seiten parallel zu der Hauptachse (19) und dessen

längere Seiten rechtwinklig zu der Hauptachse (19) verlaufen, wobei die zur Hauptausdehnungsebene (37) rechtwinkelige Normalenachse (38) der Membraneinheit (33) winkelig zu der Hauptachse (19) verläuft, wobei zweckmäßigerweise die Hauptausdehnungsebene (37) der Membraneinheit (33) zwischen zwei sich diagonal gegenüberliegenden Eckbereichen des rechteckigen Querschnittes der Gehäusekammer (17) verläuft.

14. Sortiervorrichtung nach Anspruch 12 oder 13, **dadurch gekennzeichnet, dass** die der Arbeitskammer (34) entgegengesetzte Teilkammer (36) der Gehäusekammer (17) eine Anschlusskammer (53) bildet, in der sich die bezüglich der Membraneinheit (33) getroffenen elektrischen Anschlussmaßnahmen (52a, 52b) befinden, wobei die Arbeitskammer (34) keine derartigen elektrischen Anschlussmaßnahmen enthält.

15. Sortiervorrichtung nach einem der Ansprüche 5 bis 14, **dadurch gekennzeichnet, dass** die Ausstoßöffnung (32) quer zur Richtung der Ausstoßbewegung (43) der Membraneinheit (33) orientiert ist, und/oder dass die auf eine Kreisfläche umgerechnete Fläche der Verdrängungsmembran (47) den fünffachen bis zwanzigfachen Durchmesser der einen kreisförmigen Querschnitt aufweisenden Ausstoßöffnung (32) aufweist, und/oder dass der Durchmesser "D" der einen kreisförmigen Querschnitt aufweisenden Ausstoßöffnung (32) die Bedingung

$$D = a \cdot V^{1/3}$$

erfüllt, wobei "a" ein Zahlenwert zwischen 0,3 und 3 ist und "V" das bei Aktivierung der Membraneinheit (33) aus der Arbeitskammer (34) verdrängte Volumen ist, und/oder dass das Generatorgehäuse (16) außen Flachgestalt aufweist und in Richtung einer Hauptachse (19) geringere Außenabmessungen besitzt als rechtwinkelig dazu, und/oder dass die Arbeitskammer (34) von mehreren gleichzeitig und gleichsinnig aktivierbaren Membraneinheiten (33) begrenzt ist, wobei sie zweckmäßigerweise zwischen zwei sich gegenüberliegenden und hierbei unter Einschluss eines spitzen Winkels geneigt zueinander angeordneten Membraneinheiten (33) angeordnet ist.

16. Sortiervorrichtung nach einem der Ansprüche 5 bis 15, **dadurch gekennzeichnet, dass** sie eine elektronische Steuereinrichtung (7) zur Aktivierung der mindestens einen Membraneinheit (33) des mindestens einen Luftstoßgenerators (8) aufweist, wobei sie zweckmäßigerweise eine zur Überprüfung der zu sortierenden Teile (2) dienende Detektionseinheit (6) aufweist, die an die elektronische Steuereinrichtung (7) angeschlossen ist.

17. Sortiervorrichtung nach einem der Ansprüche 5 bis 16, **dadurch gekennzeichnet, dass** sie mindestens ein sich aus mehreren, in einer Aufreihungsrichtung (15) aneinandergesetzten Luftstoßgeneratoren (8) zusammensetzendes Generatorpaket (14) aufweist, wobei die Ausstoßöffnungen (32) sämtlicher Luftstoßgeneratoren (8) zweckmäßigerweise identisch ausgerichtet sind.

## Claims

1. Method for sorting out parts (2) by means of air blasts, **characterised in that** the air blasts directed onto the parts (2) to be sorted out are generated in the form of toroidal vortices.

2. Method according to claim 1, **characterised in that** the air for generating the vortex-type air blasts is pressed in a pulsed manner through an ejection opening (32) having a circular cross-section.

3. Method according to claim 2, **characterised in that** the diameter of the ejection opening (32) is at least half as large as the smallest dimension of the part (2) to be sorted by means of applying an air blast.

4. Method according to any of claims 1 to 3, **characterised in that** at least one diaphragm unit (33) having a flexible displacement diaphragm (47) is selected using in particular electric activating impulses in such a way that its deflection resulting from this effects a pulsed ejection of air from an operating chamber (34) through the ejection opening (32), which is caused by the active displacement of air.

5. Sorting device sorting out parts (2) by means of air blasts, comprising at least one air blast generator (8) having a generator housing (16) in which at least one reversibly deflectable diaphragm unit (33) bounds an operating chamber (34) connected to at least one ejection opening (32), wherein the ejection of an air blast from the operating chamber (34) through the ejection opening (32) can be caused by the activation of the diaphragm unit (33), wherein the diaphragm unit (33) forms an air displacement unit which contains a flexible displacement diaphragm (47) and which can be deflected by activation in terms of a sudden reduction of the volume of the operating chamber (34) in order to perform an ejection movement (43), so that it actively displaces the air forming the air blast from the operating chamber (34) through the ejection opening (32) in a pulsed manner, **characterised in that** the ejection opening (32) is designed such that it gives the ejected air blast the form of a rotating toroidal vortex.

6. Sorting device according to claim 5, **characterised in that** the diaphragm unit (33) and the ejection opening (32) are arranged such that, irrespective of the activation state of the diaphragm unit (33), there is always an open connection between the operating chamber (34) and the ejection opening (32).

7. Sorting device according to claim 5 or 6, **characterised in that** the ejection opening (32) allows air to flow through in both directions and simultaneously forms an intake opening through which air flows or is drawn into the operating chamber (34) from the outside if the diaphragm unit (33) performs a return movement opposed to the ejection movement (32).

8. Sorting device according to any of claims 5 to 7, **characterised in that** precisely one ejection opening (32) terminating at the operating chamber (34) is provided.

9. Sorting device according to any of claims 5 to 8, **characterised in that** the ejection opening (32) has a sharp-edged discharge orifice (46).

10. Sorting device according to any of claims 5 to 9, **characterised in that** the at least one diaphragm unit (33) is designed for electric activation, expediently having a piezoelectric structure.

11. Sorting device according to claim 10, **characterised in that** the diaphragm unit (33) comprises a spring-elastic displacement diaphragm (47) supported on the generator housing (16) with its outer edge section (42) and a piezoelectric transducer (48) permanently connected to the displacement diaphragm (47), wherein the displacement diaphragm (47) is preferably made of metal and expediently forms an electrode of the piezoelectric transducer (48).

12. Sorting device according to any of claims 5 to 11, **characterised in that** the displacement diaphragm (47) is arranged in an inclined orientation and in particular diagonally in the interior of a housing chamber (17) of the generator housing (16), dividing the housing chamber (17) in a gas-tight manner into a first sub-chamber (35) forming the operating chamber (34) and a second sub-chamber (36), wherein the second sub-chamber (36) is expediently used for measures designed for driving the diaphragm unit (33).

13. Sorting device according to any of claims 5 to 12, **characterised in that** the generator housing (16) encloses a housing chamber (17), the dimensions of which in the axial direction of a main axis (19) are smaller than in the direction perpendicular thereto, wherein the diaphragm unit (33) is arranged in the housing chamber (17) with a main dimensional plane (37) inclined relative to the main axis (19) and divides the housing chamber (17) into two subchambers (35, 36) of which one forms the operating chamber (34), wherein the housing chamber (17) expediently has a rectangular cross-section with shorter sides extending parallel to the main axis (19) and longer sides extending perpendicular to the main axis (19), wherein the normal axis (38) of the diaphragm unit (33), which is perpendicular to the main dimensional plane (37), extends at an angle relative to the main axis (19), the main dimensional plane (37) of the diaphragm unit (33) expediently extending between two corner regions of the rectangular cross-section of the housing chamber (17) which are diagonally opposite one another.

14. Sorting device according to claim 12 or 13, **characterised in that** the sub-chamber (36) of the housing chamber (17) which is opposite the operating chamber (34) forms a connecting chamber which accommodates the electric connection measures (52a, 52b) devised for the diaphragm unit (33), the operating chamber (34) not containing any such electric connection measures.

15. Sorting device according to any of claims 5 to 14, **characterised in that** the ejection opening (32) is oriented transversely relative to the direction of the ejection movement (43) of the diaphragm unit (33), and/or **in that** the area of the displacement diaphragm (47), when converted to a circular area, has a diameter which is five to twenty times that of the ejection opening (32) with its circular cross-section, and/or **in that** the diameter "D" of the ejection opening (32) with its circular cross-section meets the condition

$$D = a \bullet V^{1/3}$$

wherein "a" is a numerical value between 0.3 and 3 and "V" is the volume displaced from the operating chamber (34) on activation of the diaphragm unit (33), and/or **in that** the generator housing (16) has a flat shape on the outside and smaller external dimensions in the direction of a main axis (19) than perpendicular thereto, and/or **in that** the operating chamber (34) is bounded by a plurality of diaphragm units (33) capable of being activated simultaneously and in the same direction, wherein it is expediently located between two opposite diaphragm units (33) which are inclined relative to one another while enclosing an acute angle.

16. Sorting device according to any of claims 5 to 15, **characterised in that** it comprises an electronic control unit (7) for activating the at least one diaphragm unit (33) of the at least one air blast generator (8), wherein it expediently comprises a detection unit (6) for checking the parts (2) to be sorted, which is connected to the electronic control unit (7).

17. Sorting device according to any of claims 5 to 16, **characterised in that** it comprises a generator set (14) made up from a plurality of air blast generators (8) arranged in a line-up direction (15), wherein the ejection openings (32) of all air blast generators (8) are expediently oriented identically.

**Revendications**

1. Procédé de tri de pièces (2) par jets d'air, **caractérisé en ce qu'**on génère les jets d'air dirigés vers les pièces (2) à trier sous forme de tourbillons toroïdaux.

2. Procédé selon la revendication 1, **caractérisé en ce qu'**on presse l'air par impulsion pour générer les jets d'air en tourbillons par une ouverture de rejet (32) présentant une section transversale circulaire.

3. Procédé selon la revendication 2, **caractérisé en ce que** le diamètre de l'ouverture de rejet (32) est au moins moitié moins grand qu'une plus petite dimension de la pièce (2) à trier respectivement par alimentation par un jet d'air.

4. Procédé selon l'une quelconque des revendications 1 à 3, **caractérisé en ce qu'**on commande une unité de membrane (33) présentant une membrane de déplacement flexible (47) avec des impulsions d'activation en particulier électriques de telle manière que sa déviation en résultant provoque un rejet d'air par impulsion suscité par le déplacement actif de l'air hors d'une chambre de travail (34) au travers d'une ouverture de rejet (32).

5. Dispositif de tri pour le tri de pièces (2) par jets d'air, avec au moins un générateur de jet d'air (8) qui présente un boîtier de générateur (16), dans lequel au moins une unité de membrane (33) déviable de manière réversible délimite une chambre de travail (34) reliée à au moins une ouverture de rejet (32), la sortie d'un jet d'air de la chambre de travail (34) au travers de l'ouverture de rejet (32) pouvant être suscitée par l'activation de l'unité de membrane (33), l'unité de membrane (33) formant une unité de déplacement d'air contenant une membrane de déplacement flexible (47), qui peut être déviée par son activation au sens d'une diminution brusque du volume de la chambre de travail (34) pour la réalisation d'un mouvement de rejet (43) de sorte qu'elle déplace activement par impulsion l'air formant le jet d'air au travers de l'ouverture de rejet (32) hors de la chambre de travail (34), **caractérisé en ce que** l'ouverture de rejet (32) est conçue de sorte à réaliser le jet d'air sortant comme un tourbillon d'air (vortex) toroïdal rotatif en lui-même.

**6.** Dispositif de tri selon la revendication 5, **caractérisé en ce que** l'unité de membrane (33) et l'ouverture de rejet (32) sont disposées de telle manière qu'indépendamment de l'état d'activation de l'unité de membrane (33), une liaison toujours ouverte entre la chambre de travail (34) et l'ouverture de rejet (32) est établie.

**7.** Dispositif de tri selon la revendication 5 ou 6, **caractérisé en ce que** l'ouverture de rejet (32) permet un passage d'air dans les deux directions et forme en même temps une ouverture d'aspiration, par laquelle de l'air entre ou est aspiré de l'extérieur dans la chambre de travail (34), si l'unité de membrane (33) réalise un mouvement de rappel dans le sens inverse au mouvement de rejet (34).

**8.** Dispositif de tri selon l'une quelconque des revendications 5 à 7, **caractérisé en ce qu'**une ouverture de rejet (32) débouchant dans la chambre de travail (34) est précisément présente.

**9.** Dispositif de tri selon l'une quelconque des revendications 5 à 8, **caractérisé en ce que** l'ouverture de rejet (32) présente une ouverture de sortie (46) à arêtes vives.

**10.** Dispositif de tri selon l'une quelconque des revendications 5 à 9, **caractérisé en ce que** l'au moins une unité de membrane (33) est réalisée de sorte à être activable électriquement, celle-ci possédant de manière appropriée une structure piézoélectrique.

**11.** Dispositif de tri selon la revendication 10, **caractérisé en ce que** l'unité de membrane (33) présente une membrane de déplacement (47) sollicitée par ressort en appui avec sa section de bord extérieure (42) contre le boîtier de générateur (16) ainsi qu'au moins un piézoconvertisseur (48) relié à la membrane de déplacement (47), la membrane de déplacement (47) se composant de préférence de métal et formant de manière appropriée en même temps une électrode du piézoconvertisseur (48).

**12.** Dispositif de tri selon l'une quelconque des revendications 5 à 11, **caractérisé en ce que** la membrane de déplacement (47) est disposée avec une orientation en biais et en particulier en diagonale à l'intérieur d'une chambre (17) du boîtier de générateur (16), celle-ci divisant de manière étanche au gaz la chambre de boîtier (17) en une première chambre partielle (35) formant la chambre de travail (34) ainsi qu'en une seconde chambre partielle (36), la seconde chambre partielle (36) étant utilisée de manière appropriée pour des mesures servant à l'entraînement de l'unité de membrane (33).

**13.** Dispositif de tri selon l'une quelconque des revendications 5 à 12, **caractérisé en ce que** le boîtier de générateur (16) entoure une chambre de boîtier (17), dont les dimensions sont moindres dans le sens axial d'un axe principal (19) que dans le sens orthogonal à celui-ci, l'unité de membrane (33) étant disposée dans la chambre de boîtier (17) avec un plan d'extension principal (37) incliné par rapport à l'axe principal (19) et divisant la chambre de boîtier (17) en deux chambres partielles (35, 36), dont l'une forme la chambre de travail (34), la chambre de boîtier (17) présentant de manière appropriée une section transversale rectangulaire, dont les petits côtés s'étendent parallèlement à l'axe principal (19) et les grands côtés perpendiculairement à l'axe principal (19), l'axe normal (38) orthogonal au plan d'extension principal (37) de l'unité de membrane (33) s'étendant de manière angulaire à l'axe principal (19), le plan d'extension principal (37) de l'unité de membrane (33) s'étendant de manière appropriée entre deux zones de coin en regard en diagonale de la section transversale rectangulaire de la chambre de boîtier (17).

**14.** Dispositif de tri selon la revendication 12 ou 13, **caractérisé en ce que** la chambre partielle (36) en regard de la chambre de travail (34) de la chambre de boîtier (17) forme une chambre de raccordement (53), dans laquelle les mesures de raccordement (52a, 52b) électriques prises par rapport à l'unité de membrane (33) se trouvent, la chambre de travail (34) ne contenant aucune mesure de raccordement électrique de la sorte.

**15.** Dispositif de tri selon l'une quelconque des revendications 5 à 14, **caractérisé en ce que** l'ouverture de rejet (32) est orientée transversalement à la direction du mouvement de rejet (43) de l'unité de membrane (33) et/ou **en ce que** la surface calculée sur une surface circulaire de la membrane de déplacement (47) présente le quintuple à vingtuple diamètre de l'ouverture de rejet (32) présentant une section transversale circulaire, et/ou **en ce que** le diamètre « D » de l'ouverture de rejet (32) présentant une section transversale circulaire satisfait à la condition

$$D = a \cdot V^{1/3}$$

« a » étant une valeur numérique comprise entre 0,3 et 3 et « V » étant le volume déplacé lors de l'activation de l'unité de membrane (33) hors de la chambre de travail (34) et/ou **en ce que** le boîtier de générateur (16) présente à l'extérieur une forme plate et possède en direction d'un axe principal (19) de moindres dimensions extérieures qu'orthogonalement à celui-ci, et/ou **en ce que** la chambre de travail (34) est délimitée par plusieurs unités de membrane (33) activables en même temps et dans le même sens, celle-ci étant disposée de manière appropriée entre deux unités de membrane (33) en regard et disposées en même temps en formant un angle aigu de manière inclinée l'une par rapport à l'autre.

16. Dispositif de tri selon l'une quelconque des revendications 5 à 15, **caractérisé en ce qu'**il présente un dispositif de commande électronique (7) pour l'activation de l'au moins une unité de membrane (33) d'au moins un générateur de jet d'air (8), celle-ci présentant de manière appropriée une unité de détection (6) servant à la vérification des pièces (2) à trier qui est raccordée au dispositif de commande (7) électronique.

17. Dispositif de tri selon l'une quelconque des revendications 5 à 16, **caractérisé en ce qu'**il présente au moins un paquet de générateurs (14) composé de plusieurs générateurs de jet d'air (8) placés les uns sur les autres dans un sens d'alignement (15), les ouvertures de rejet (32) de tous les générateurs de jet d'air (8) étant orientées de manière appropriée et identique.

Fig. 1

Fig. 5

Fig. 3

Fig. 2

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5628411 A
- DE 4030344 A1
- DE 3808798 A1
- DE 202007001884 U1
- WO 2006083635 A2
- DE 4033081 A